(19) Europäisches Patentamt
European Patent Office
Office européen des brevets

(11) Publication number: **0 096 457**
**B1**

(12) **EUROPEAN PATENT SPECIFICATION**

(45) Date of publication of patent specification: **11.03.87**

(51) Int. Cl.$^4$: **C 07 D 215/30,** A 61 K 31/555

(21) Application number: **83302243.7**

(22) Date of filing: **20.04.83**

# (54) Indium complexes of substituted 8-hydroxy-quinolines, pharmaceutical compositions containing them and their use as antibacterial agents.

(30) Priority: **22.04.82 GB 8211680**
**14.09.82 GB 8226157**

(43) Date of publication of application:
**21.12.83 Bulletin 83/51**

(45) Publication of the grant of the patent:
**11.03.87 Bulletin 87/11**

(84) Designated Contracting States:
**BE CH DE FR GB IT LI NL SE**

(56) References cited:
**Chemical Abstracts vol. 74, no. 26, 28 June 1971, Columbus, Ohio, USA C.A. NGUYEN et al. "Extraction-photometric determination of indium and gallium using 5,7-dibromohydroxyquinoline", page 654, abstract no. 150807k**

**JOURNAL OF MEDICINAL CHEMISTRY, vol. 13, no. 5, 1970, Washington A.O. FITTON et al. "Synthesis and antimicrobial activity of 5,7-dichloroquinoline-8-thiol and its derivatives", pages 1008-1009**

(73) Proprietor: **NATIONAL RESEARCH DEVELOPMENT CORPORATION**
**101 Newington Causeway**
**London SE1 6BU (GB)**

(72) Inventor: **Rogers, Henry Joseph**
**6 High Street**
**Clophill Bedford (GB)**

(74) Representative: **Stephenson, Gerald Frederick**
**Patent Department**
**National Research Development Corporation**
**101 Newington Causeway**
**London SE1 6BU (GB)**

(56) References cited:
**Chemical Abstracts vol. 94, no. 4, 26 January 1981, Columbus, Ohio, USA M. RAMAIAH et al. "A polarographic study of indium complexes with sulfoxine (8-hydroxyquinoline-5-sulfonic acid)", page 438, abstract no. 22136s**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 096 457 B1

**Description**

This invention relates to compounds having pharmaceutical activity, particularly as antibacterial agents.

8-Hydroxyquinoline and certain derivatives thereof have been used as pharmaceuticals, particularly by virtue of their anti-bacterial action, and especially against gram-positive bacteria. This gram-positive anti-bacterial activity is reported to require the presence of one of the cation $Cu^{2+}$, $Fe^{2+}$ and $Fe^{3+}$, and it is believed that the active antibacterial moiety is a complex formed between such a metal cation and the 8-hydroxyquinoline or its derivative [Selective Toxicity by Adrien Albert (Methuen), Fourth Edition, 1968, pages 331 to 343]. 8-Hydroxyquinoline and derivatives thereof have also been employed for the treatment of parasitic and fungal infections. Usually, however, 8-hydroxyquinoline and its derivatives have been limited to topical use, for example for the treatment of dermatoses, although some oral use has occurred in the case of parasitic infections, for example for the treatment of amoebiasis, since parenteral use of the compounds has been ruled out due to their inactivation by serum. Furthermore, the use of the compounds as antibiotics has hitherto largely been limited to the treatment of gram-positive infections.

Despite this somewhat restricted picture of the value of 8-hydroxyquinoline derivatives as pharmaceuticals and the reported necessity for the presence of $Cu^{2+}$, $Fe^{2+}$ or $Fe^{3+}$ in order for their gram-positive activity to be expressed, the activity has been investigated of complexes of 8-hydoxyquinoline derivatives with metals other than iron or copper and it has been found that the indium complexes of certain halogenated 8-hydroxyquinolines show considerable promise as antibacterial agents without the limitations referred to above. Not only do the complexes show a markedly higher antibacterial activity than the corresponding metal-free 8-hydroxyquinoline compounds, but they show good levels of activity against gram-negative bacteria and, moreover, has shown activity when given parenterally *in vivo*.

Although indium (III) complexes of 5-chloro, 5,7-dichloro-5-chloro-7-iodo- and 5,7-dibromo-8-hydroxyquinoline have been disclosed [Rane, Indian Journal of Chemistry, 1981, *20A,* 900; Miura *et al*, Journal of Chromatography, 1981, *210*, 536; Nguyen and Zharovskii, Urkrainskii Khimisheskii Zhurnal, 1969, *35*, 1321, and 1970, *36*, 947 and 1273 (Chemical Abstracts, 1970, *72*, 71192m, and 1971, *74*, 35223e and 150807k)] these reports deal only with physical properties of the complexes such as their solubility and spectral characteristics, and in all cases except one the complexes were prepared in solution, the only solid complex being that of 5,7-dibromo-8-hydroxyquinoline reported by Nguyen and Zharovskii (Ukr. Khim. Zh., 1970, *36*, 947).

Accordingly, the present invention comprises a complex with indium (III) of a ring substituted derivative of 8-hydroxyquinoline which has a halogen substituent at one or both of the 5- and 7-positions and/or a halogenated $C_{1-4}$ alkyl group substituent at the 2-position, and which may optionally have one or more additional ring substituents selected from the group consisting of $C_{1-4}$ alkyl, carboxy and sulpho and physiologically acceptable caboxylate and sulphonate salts, for use in therapy.

The complexes may contain various proportions of indium and 8-hydroxyquinoline derivative. In one form of complex, the preparation of which in solution is described in Example 1, there is a 1:2 molar proportion of indium:8-hydroxyquinoline derivative. In such a complex, the charge on the trivalent indium (III) cation, $In^{3+}$, will be only partially neutralised by the anions derived from the 8-hydroxy group of each molecule of the derivative through the reaction $OH \rightarrow O^-$ which occurs on formation of the complex, and an additional monovalent physiologically acceptable anion is required to establish neutrality. Such an anion may, for example, be a halide ion such as the chloride ion or may be any other such physiologically acceptable anion, for example one selected with a particular view to enhancing the aqueous solubility of the complex and being derived from an acid such as methane sulphonic acid, isethionic acid, etc. Other forms of complex are those containing a 1:1 molar proportion of indium:8-hydroxyquinoline derivative, which require a divalent or two monovalent physiologically acceptable anions to establish neutrality, and those containing a 1:3 molar proportion of indium:8-hydroxyquinoline derivative, such 1:3 complexes having the advantage of being neutral as such without the presence of an additional anion.

The present invention does not, however, exclude complexes containing other proportions of indium:8-hydroxyquinoline derivative. Spectrometric studies of the stoichiometry of the reaction of $In^{3+}$ with various 8-hydroxyquinolne derivatives have indicated the formation in solution of complexes containing various stoichiometries so that with 7-iodo-8-hydroxyquinoline 5-sulphonic acid, for example, the formation of a 1:5 complex is indicated.

Among the halogens comprising a halogen substituent, iodo and especially chloro groups are of somewhat greater interest, although fluoro and especially bromo groups are also of interest. Halogen substitution at the 5-position, either as a 5,7-dihalogenated derivative or as a 5-monohalogenated derivative is often of most interest. In the case of the halogenated alkyl group substituents, fluorinated groups are of particular interest and the halogenated alkyl group is preferably a halogenated methyl group such as a trifluoromethyl group.

The 8-hydroxyquinoline derivative may contain two or more different halogen atoms, and may contain both a 5- and/or 7-halogen substituent and a 2-halogenated alkyl group substituent, although halogen substitution is in general of greater interest. Other types of substituent may also be present, either at one of the 5- or 7-positions and/or at other ring carbon atom positions, including the 2-position, although it is possible that substitution adjacent to the nitrogen atom may inhibit the formation of the usual form of

complex and result instead in the formation of an alternative form of complex, for example containing a 1:6 proportion of indium:8-hydroxyquinoline derivative, which may possess a lower level of activity. For this reason, 2-substituted 8-hydroxyquinoline derivatives, including those having a halogenated alkyl group substituent at this position, are of somewhat lesser interest in the context of the present invention than derivatives not containing such a substituent.

Additional substituents may include one or more $C_{1-4}$ alkyl groups such as methyl, for example a 2-methyl group, but groups of particular interest are those of an ionic nature which confer a greater degree of water solubility on the derivative, although it should be appreciated that the presence of an additional substituent may also reduce activity, for example as discussed above. Such ionic groups include carboxylic and sulphonic acid groups in either the free acid form ($—CO_2H$ and $—SO_3H$) or in the form of physiologically acceptable salts such as those formed with the alkali metals, for example sodium, or with an ammonium or substituted ammonium cation. Other opportunities for enhancing water solubility through salt formation are somewhat limited since the hydroxy group of each 8-hydroxyquinoline derivative molecule in the complex is usually involved in complex formation and involvement of the nitrogen lone pair of electrons in salt formation can adversely affect complex formation.

Examples of specific complexes of use in the present invention are the indium (III) complexes of 5-chloro-8-hydroxyquinoline, 7-chloro-8-hydroxyquinoline, 5,7-dichloro-8-hydroxyquinoline, 5-chloro-7-iodo-8-hydroxyquinoline, 5,7-diiodo-8-hydroxyquinoline, 5,7-dibromo-8-hydroxyquinoline, 7-bromo-5-chloro-8-hydroxyquinoline, and 7-iodo-8-hydroxyquinoline 5-sulphonic acid and physiologically acceptable salts thereof formed with the sulphonic acid group. The indium (III) complexes of the 2-methyl derivatives of these compounds, for example 5,7-dichloro-2-methyl-8-hydroxyquinoline, may also be specifically mentioned, as may the indium complexes of 2-trifluoromethyl-8-hydroxyquinoline and of the analogues of the 2-methyl compounds referred to above in which the 2-methyl group is replaced by a 2-trifluoromethyl group.

It will be appreciated that the majority of the complexes described herein are novel and that these novel complexes, *per se*, are included by the present invention.

The complexes are conveniently prepared by reaction in a suitable mutual solvent of the 8-hydroxyquinoline derivative and a suitable indium salt, for example an indium halide and particularly indium chloride, usually as the trihydrate, $InCl_3 \cdot 3H_2O$, in a selected proportion, for example a 1:2 molar proportion of salt:derivative. Where the complex contains an additional anion to achieve neutrality, the indium salt may itself provide this anion, although it is possible if desired to insert an alternative anion subsequent to the initial formation of a complex. Suitable solvents for the reaction of derivative and indium salt include organic solvents such as alcohols and ketones, indium chloride being of interest in this respect for its significant level of solubility in oraganic solvents. Alternatively, aqueous solvent mixtures may be used so that in one procedure, for example, the indium salt in dilute aqueous hydrochloric acid is mixed with a solution of the 8-hydroxyquinoline derivative in ethanol, or in dimethylsulphoxide. Reaction to form the complex usually takes place quite rapidly at room temperature so that a period of 30 minutes, for example, is usually fully sufficient. Depending upon the solvent used, the particular reactants and their concentration, precipitation may occur from the reaction solution or the solvent may be removed *in vacuo* to leave the complex. An alternative procedure for isolation of the complex, which is illustrated in Example 2 for 5,7-dichloro-8-hydroxyquinoline, involves dilution of a solution of the complex in an organic solvent such as ethanol or dimethyl sulphoxide with water.

Synthetic routes to a variety of 5- and/or 7-halogenated 8-hydroxyquinolines are described in the literature. 2-Trifluoromethyl-8-hydroxyquinoline may be prepared by the procedure of March *et al*, Journal of Medicinal Chemistry, 1973, *16*, 337—342 and other derivatives containing a 2-trifluoromethyl group by an analogous procedure. It will be appreciated that, in general, modifications of the procedures described in the art for the preparation of the 8-hydroxyquinoline derivatives and of the procedures described above for the preparation of the indium complexes may be used, if desired, as will be apparent to those skilled in the art.

The formulation of complexes according to the present invention for use as a pharmaceutical for both human and animal admiministration may be effected by a variety of methods, but usually involves the use of a physiologically acceptable diluent or carrier. The complex may, for instance, be applied as an aqueous or oily solution, suspension or emulsion for parenteral administration, the composition therefore preferably being sterile and pyrogen-free. The complex may also be compounded for oral administration, more usually in the presence of conventional solid carrier materials such as starch, lactose, dextrin and magnesium stearate. Alternative formulations are as aerosols, suppositories, cachets and, for localised treatment, as suitable creams, lotions or drops.

The compositions may conveniently be formulated in unit dosage form, i.e. in the form of discrete portions each containing a unit dose, or a multiple or sub-multiple of a unit dose. Dosage levels may, however, vary quite considerably according to the particular complex and also the type of treatment in which they are used but, as a general guide it may be stated that a suitable regimen, particularly as regards systemic treatments, often involves daily doses at a level of 1 to 20 mg/kg, for example 2.5 or 5 to 10 mg/kg, extending over a suitable period, for example 5—10 days, and repeated, often after a rest period, as required. It will be appreciated, however, that in some circumstances higher or lower dose levels than this may be appropriate.

Compositions containing complexes according to the present invention are of interest for the treatment of a range of bacterial infections. Thus, the complexes are of interest for their activity against gram-positive bacteria, in particular those belonging to the genera *Staphylococcus* and *Strephtococcus. In vitro* experiments with *Staphylococcus aureus* and *Staphylocuccus epidermidis* have shown a markedly higher level of activity for the indium complexes than for the corresponding metal free derivative of 8-hydroxyquinolone. Moreover, the complexes show very worthwhile activity against gram-negative bacteria, especially aerobic rather than anaerobic bacteria, for example various *Enterobacteriaceae* such as *Escherichia, Klebsiella, Aerobacter* and *Salmonella*. It will be appreciated, however, that the complexes according to the present invention and specific complexes in particular, may be of more interest in the treatment of infections caused by some of these bacteria than by some others. Thus, for example, we have found that in general the level of activity of the complexes against *Klebsiella pneumoniae, Escherichia coli* and *Salmonella typhinurium* is often greater than against *Pseudomonas aeruginosa.*

In addition to systemic use, including parental administration for which the indium complexes, unlike the metal-free compounds, are suitable, the complexes are also of interest for localised action, for example through topical application to the skin for the treatment of gram-negative and especially gram-positive bacterial infections, for example for the treatment of seborrheic dermatitis, the higher activity of the indium complexes giving them a marked advantage as compared with the metal-free compounds in this area also. As indicated previously, formulations for topical administration include creams, lotions, etc. of use, for example, as an ointment or particularly as a shampoo. Other types of formulation for topical adminstration include a formed material, for example in the form of a patch, etc., which may be attached to the skin at a particular place on the body and thereby provide a dose of the indium complex at that location by release from the material.

An alternative use, of particular relevance in the context of gram-negative bacterial infections and where treatment may be effected at the required site through oral administration, is the treatment of infections of the gut, for example the treatment of gastroenteritis caused by *E. coli,* typhoid fever, chloera caused by *Vibrio cholerae* and dysentery caused by *Shigella.*

Although the compounds of the present ivnention are of particular interest for the treatment of bacterial infections, they are also of some interest for the treatment of fungal and especially parasitic infections, being used in a broadly similar manner to that described above for the treatment of bacterial infections.

The invention is illustrated by the following Examples.

EXAMPLES

Example 1: Preparation of Indium Complexes

1:2 complexes are prepared in solution of $In^{3+}$ with the 8-hydroxyquinoline derivatives: 5-chloro-8-hydroxyquinoline; 5,7-dichloro-8-hydroxyquinoline; 5-chloro-7-iodo-8-hydroxyquinoline; and 5,7-diiodo-8-hydroxyquinoline. In each case indium chloride trihydrate ($InCl_3 \cdot 3H_2O$; 0.137 g 0.5 m.mole) and 1 m.mole of the 8-hydroxyquinoline derivative are dissolved together in dimethylsulphoxide (20 ml) to give a 0.5 mM solution of the complex, the formula of which is illustrated below in respect of the second of the 8-hydroxyquinoline derivatives named above.

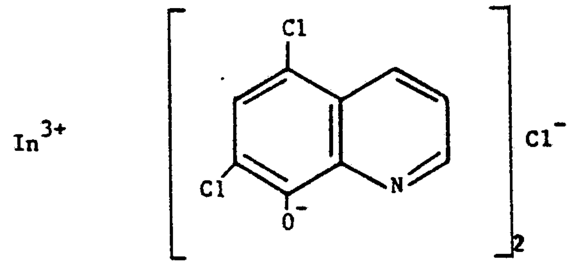

Example 2: Preparation of Indium Complex of 5,7-Dichloro-8-Hydroxyquinoline

Indium chloride trihydrate (1.37 g, 5.0 m.mole) and 5,7-dichloro-8-hydroxyquinoline (2.14 g, 10 m.mole) are dissolved together in ethanol (200 ml) and the solution is concentrated to dryness on a rotary evaporator. The resulting yellow glass is recrystallised by solution in a small volume of ethanol followed by the careful addition of water to give an indium complex of 5,7-dichloro-8-hydroxyquinoline as a bright yellow powder with a melting point above 340°C. The complex has a low solubility in water but is readily soluble in a wide range of organic solvents.

Example 3: In Vitro Activity of Indium Complexes

Experiments were carried out to find the minimal inhibitory concentrations (MIC) for solutions of various indium complexes against *Staphylococcus aureus* (human isolate), *Escherichia coli* (018; co1V$^+$) and *Klebsiella pneumoniae* and to compare these with the MIC values for the corresponding metal-free

compounds. The complexes were prepared in solution as described in Example 1 except that in the case of the complex of 7-iodo-8-hydroxyquinoline 5-sulphonic acid, 5 molar equivalents of metal-free compound were reacted with the indium chloride trihydrate and the solution was prepared in water. The solutions of the metal-free compounds were prepared in the same solvent as used for the equivalent complex.

The culture medium used consisted of a 9:1 mixture of trypticase soy broth and 6% w/v aqueous sodium bicarbonate solution. The medium was bulk inoculated with approximately $10^4$ test bacteria per ml and 5.0 ml aliquots were then dispensed into sterile 20 ml bottles to which 0.05 ml of the test solution had been added. The test solutions were obtained from the original solution of the indium complex or metal-free compound by dilution to give a suitable range of concentrations in µM. Control samples, to which either no test solution was added or to which the solvent of the test solution only was added, were also prepared.

The bottles were incubated on a rotary shaker for 18 hours at 37°C in an atmosphere of 5% $CO_2$ in air (higher MIC values being obtained if the sodium bicarbonate and the $CO_2$ were omitted). For each compound tested the samples at the various concentrations were studied to determine the lowest concentration of the compound at which no growth could be seen. The MIC values thus obtained are shown in Table 1 where it will be seen that the values are in each case lower for the indium complex as compared with the metal-free compound. (The controls showed that the solvents had no obvious effect on bacterial growth).

TABLE 1

| Compound | MIC Values (µM) | | |
|---|---|---|---|
| | *S. aureus* | *E. coli* | *K. pneumoniae* |
| 5,7-dichloro-8-hydroxyquinoline | 6.3 | 100 | 100 |
| Indium complex | 3.2 | 1.6 | 12.5 |
| 5-Chloro-8-hydroxyquinoline | 18.8 | 38.5 | 38.5 |
| Indium complex | 1.6 | 6.3 | 25 |
| 7-Iodo-8-hydroxyquinoline 5-sulphonic acid | >500 | >500 | >500 |
| Indium complex | 50 | >100 | >100 |

Generally similar results have been obtained with *Staphylococcus epidermis* (human isolate) as for *S. aureus*.

Example 4: In Vivo Tests of Indium Complex of 5,7-Dichloro-8-Hydroxyquinoline

Experiments were carried out to compare the activity *in vivo* against *E. coli* (108, co1V$^+$), *K. pneumoniae* and *S. aureus* (human isolate) of the indium complex of 5,7-dichloro-8-hydroxyquinoline with that of the metal-free compound.

The solutions for administration were prepared as follows:

(1) 2 equivalents of 5,7-dichloro-8-hydroxyquinoline were dissolved in a 15 mM solution of $InCl_3 \cdot 3H_2O$ in ethanol containing 1% v/v of glycerol. A 1.0 ml aliquot of this solution was dried *in vacuo* using a sterile universal container and the resultant residue was treated with 15 ml of sterile 0.15 M aqueous sodium chloride containing 1% w/v of sodium bicarbonate and the solid dispersed by sonication. A 0.2 ml dose of the final preparation corresponds to a dose of 4.6 mg/kg in a 25 g mouse.

(2) 6.4 mg of 5,7-dichloro-8-hydroxyquinoline were moistened by the addition of 0.05 ml of dimethyl sulphoxide and to the moistened material was added 15 ml of sterile 0.15 M aqueous sodium chloride containing 1% w/v of sodium bicarbonate, the solid then being dispersed by sonication. A 0.2 ml dose of the final preparation corresponds to a dose of 3.4 mg/kg in a 25 g mouse.

Groups of 5 male or female Parks mice weighing from 23—25 g were infected by intraperitoneal injection of one of $8.0 \times 10^6$ *E. coli,* $3.5 \times 10^5$ *K. pneumoniae* and $7.0 \times 10^8$ *S. aureus,* the organisms in each case being suspended in 0.2 ml of 10% v/v trypticase soy broth in 0.15 M aqueous sodium chloride, three groups of mice being infected with each organism. For each organism, one of the three groups of infected mice was treated by intraperitoneal injection with 0.2 ml of a suspension of the indium complex whilst a second group was similarly treated with 0.2 ml of a supsension of the metal-free compound. The treatment was given twice per day for 1 day in the case of *E. coli* infection, twice per day for 4 days in the case of *K. pneumoniae* infection, and twice per day for 2 days in the case of the *S. aureus* infection. The third group of mice in each case received no treatment.

The results obtained in terms of the number of deaths at 7 days and the percentage of deaths in the group are shown in Table 2 where it will be seen that in each case the indium complex showed a much greater anti-bacterial effect than the metal-free compound.

TABLE 2

| Compound | Organism | Deaths at 7 days | Deaths % |
|---|---|---|---|
| Control | | 5 | 100 |
| 5,7-Dichloro-8-hydroxyquinoline | *E. coli* | 5 | 100 |
| Indium complex | | 0 | 0 |
| Control | | 5 | 100 |
| 5,7-Dichloro-8-hydroxyquinoline | *K. pneumoniae* | 5 | 100 |
| Indium complex | | 0 | 0 |
| Control | | 4 | 80 |
| 5,7-Dichloro-8-hydroxyquinoline | *S. aureus* | 4 | 80 |
| Indium complex | | 2 | 40 |

**Claims**

1. A complex with indium (III) of a ring substituted derivative of 8-hydroxyquinoline having a halogen substituent at one or both of the 5- and 7-positions and/or a halogenated $C_{1-4}$ alkyl group substituent at the 2-position, and which may optionally have one or more additional ring substituents selected from the group consisting of $C_{1-4}$ alkyl, carboxy and sulpho and physiologically acceptable carboxylate and sulphonate salts, for use in therapy.

2. A complex with indium (III) of a ring substituted derivative of 8-hydroxyquinoline having a halogen substituent at one or both of the 5- and 7-positions, and which may optionally have one or more additional ring substituents selected from the group consisting of $C_{1-4}$ alkyl, carboxy and sulpho and physiologically acceptable carboxylate and sulphonate salts, for use in therapy.

3. A complex with indium (III) of a ring substituted derivative of 8-hydroxyquinoline having a halogen substituent at one or both of the 5- and 7-positions and/or a halogenated $C_{1-4}$ alkyl group substituent at the 2-position, and which may optionally have one or more additional ring substituents selected from the group consisting of $C_{1-4}$ alkyl, carboxy and sulpho and physiologically acceptable carboxylate and sulphonate salts, but excluding the specific 8-hydroxyquinoline derivatives, 5-chloro-8-hydroxyquinoline, 5,7-dichloro-8-hydroxyquinoline, 5-chloro-7-iodo-8-hydroxyquinoline and 5,7-dibromo-8-hydroxyquinoline.

4. A complex with indium (III) of a ring substituted derivative of 8-hydroxyquinoline having a halogen substituent at one or both of the 5- and 7-positions, and which may optionally have one or more additional ring substituents selected from the group consisting of $C_{1-4}$ alkyl, carboxy and sulpho and physiologically acceptable carboxylate and sulphonate salts, but excluding the specific 8-hydroxyquinoline derivatives, 5-chloro-8-hydroxyquinoline, 5,7-dichloro-8-hydroxyquinoline, 5-chloro-7-iodo-8-hydroxyquinoline and 5,7-dibromo-8-hydroxyquinoline.

5. A complex with indium (III) of a ring substituted derivative of 8-hydroxyquinoline having a halogen substituent at one or both of the 5- and 7-positions, and which may optionally have one or more additional ring substituents selected from the group consisting of $C_{1-4}$ alkyl, carboxy and sulpho and physiologically acceptable carboxylate and sulphonate salts, but excluding the specific 8-hydroxyquinoline derivative, 5,7-dibromo-8-hydroxyquinoline, said complex being in solid form.

6. A complex according to Claim 2, 4 or 5, in which the halogen substituent or substituents are selected from chloro, iodo and bromo.

7. A complex according to Claim 6, in which the 8-hydroxyquinoline derivative is substituted only by the halogen substituents at one or both of the 5- and 7-positions.

8. A complex according to Claim 2, in which the 8-hydroxyquinoline derivative is selected from 5-chloro-8-hydroxyquinoline, 7-chloro-8-hydroxyquinoline, 5,7-dichloro-8-hydroxyquinoline, 5-chloro-7-iodo-8-hydroxyquinoline, 5,7-diiodo-8-hydroxyquinoline, 5,7-dibromo-8-hydroxyquinoline, 7-bromo-5-chloro-8-hydroxyquinoline, and 7-iodo-8-hydroxyquinoline 5-sulphonic acid and physiologically acceptable sulphonate salts thereof.

9. A complex according to Claim 4, in which the 8-hydroxyquinoline derivative is selected from 7-chloro-8-hydroxyquinonline, 7-bromo-5-chloro-8-hydroxyquinoline, 5,7-diiodo-8-hydroxyquinoline and 7-iodo-8-hydroxyquinoline 5-sulphonic acid and physiologically acceptable sulphonate salts thereof.

10. A complex according to Claim 5 or 8, in which the 8-hydroxyquinoline derivative is 5,7-dichloro-8-hydroxyquinoline.

11. A complex according to Claim 1 or 3 which has a fluorinated $C_{1-4}$ alkyl group substituent at the 2-position.

12. A complex according to Claim 11 which has a trifluoromethyl substituent at the 2-position.

13. A complex according to Claim 12, in which the 8-hydroxyquinoline derivative is 2-trifluoromethyl-8-hydroxyquinoline.

14. A complex according to any of the preceding claims which contains a 1:1, 1:2 or 1:3 molar proportion of $In^{3+}$:8-hydroxyquinoline derivative anion.

15. A complex according to Claim 14, which contains a 1:3 molar proportion in $In^{3+}$:8-hydroxyquinoline derivative anion.

16. A process for the preparation of an indium (III) complex as defined in Claim 3 which comprises reacting the corresponding 8-hydroxyquinoline derivative with $In^{3+}$ ions.

17. A process for the preparation of an indium (III) complex as defined in Claim 5 which comprises reacting the corresponding 8-hydroxyquinoline derivative with $In^{3+}$ ions and subsequently precipitating the complex by the addition of water to an organic solvent solution thereof.

18. A process according to Claim 16 or 17, in which the $In^{3+}$ ions are provided by an indium trihalide.

19. A pharmaceutical composition which comprises an indium (III) complex of a ring substituted derivative of 8-hydroxyquinoline having a halogen substituent at one or both of the 5- and 7-positions and/or a halogenated $C_{1-4}$ alkyl group substituent at the 2-position, and which may optionally have one or more additional ring substituents selected from the group consisting of $C_{1-4}$ alkyl, carboxy and sulpho and physiologically acceptable carboxylate and sulphonate salts, together with a physiologically acceptable diluent or carrier but excluding any diluent which is non-sterile and non-pyrogen free.

20. A composition being a sterile and pyrogen-free solution, suspension or emulsion for the treatment of bacterial, fungal and parasitic infections in humans or animals which comprises an indium (III) complex as defined in Claim 19.

21. A solid composition for the treatment of bacterial, fungal or parasitic infections in humans or animals which comprises an indium (III) complex as defined in Claim 19.

22. A solid composition adapted for oral administration which comprises an indium (III) complex as defined in Claim 19.

23. A composition adapted for topical application for the treatment of bacterial, fungal and parasitic infections in humans or animals which comprises an indium (III) complex as defined in Claim 19.

24. A composition according to Claim 23, being an ointment or shampoo.

25. A composition according to Claim 23, being a formed material adapted to be retained in contact with the skin.

26. A composition according to any of Claims 19 to 25, in which the 8-hydroxyquinoline derivative has a halogen substituent at one or both of the 5- and 7-positions.

27. A composition according to Claim 26, in which the halogen substituent or substituents are selected from chloro, iodo and bromo.

28. A composition according to Claim 27, in which the 8-hydroxyquinoline derivative is substituted only by the halogen substituents at one or both of the 5- and 7-positions.

29. A composition according to Claim 27, in which the 8-hydroxyquinoline derivative is selected from 5-chloro-8-hydroxyquinoline, 7-chloro-8-hydroxyquinoline, 5,7-dichloro-8-hydroxyquinoline, 5-chloro-7-iodo-8-hydroxyquinoline, 5,7-diiodo-8-hydroxyquinoline, 5,7-dibromo-8-hydroxyquinoline, 7-bromo-5-chloro-8-hydroxyquinoline and 7-iodo-8-hydroxyquinoline 5-sulphonic acid and physiologically acceptable sulphonate salts thereof.

30. A composition according to Claim 27, in which the 8-hydroxyquinoline derivative is 5,7-dichloro-8-hydroxyquinoline.

31. The use of an indium (III) complex as defined in Claim 1 for the manufacture of a medicament of use in the treatment of bacterial, fungal and parasitic infections.

**Patentansprüche**

1. Ein Komplex mit Indium(III) eines ringsubstituierten Derivats von 8-Hydroxychinolin, das einen Halogensubstituenten in einer oder beiden der 5- und 7-Stellungen und/oder einen halogenierten $C_{1-4}$-Alkylgruppensubstituenten in 2-Stellung aufweist, und das gegebenenfalls ein oder mehrere weitere Ringsubstituenten, ausgewählt aus der Gruppe bestehend aus $C_{1-4}$-Alkyl, Carboxy und Sulfo, haben kann und die physiologisch annehmbaren Carboxylat- und Sulfonatsalze zur Verwendung in der Therapie.

2. Ein Komplex mit Indium(III) eines ringsubstituierten Derivats von 8-Hydroxychinolin, das einen Halogensubstituenten in einer oder beiden der 5- und 7-Stellungen hat und das gegebenenfalls ein oder

mehrere weitere Ringsubstituenten, ausgewählt aus der Gruppe bestehend aus $C_{1-4}$-Alkyl, Carboxy und Sulfo, haben kann und die physiologisch annehmbaren Carboxylat- und Sulfonatsalze zur Verwendung in der Therapie.

3. Ein Komplex mit Indium(III) eines ringsubstituierten Derivats von 8-Hydroxychinolin, das einen Halogensubstituenten in einer oder beiden der 5- und 7-Stellungen und/oder einen halogenierten $C_{1-4}$-Alkylgruppensubstituenten in 2-Stellung aufweist, und das gegebenenfalls ein oder mehrere weitere Ringsubstituenten, ausgewählt aus der Gruppe bestehend aus $C_{1-4}$-Alkyl, Carboxy und Sulfo, haben kann und die physiologisch annehmbaren Carboxylat- und Sulfonatsalze, jedoch ausgenommen die speziellen 8-Hydroxychinolinderivate, 5-Chlor-8-hydroxychinolin, 5,7-Dichlor-8-hydroxychinolin, 5-Chlor-7-jod-8-hydroxychinolin und 5,7-Dibrom-8-hydroxychinolin.

4. Ein Komplex mit Indium(III) eines ringsubstituierten Derivats von 8-Hydroxychinolin, das einen Halogensubstituenten in einer oder beiden der 5- und 7-Stellungen hat und das gegebenenfalls ein oder mehrere weitere Ringsubstituenten, ausgewählt aus der Gruppe bestehend aus $C_{1-4}$-Alkyl, Carboxy und Sulfo, haben kann und die physiologisch annehmbaren Carboxylat- und Sulfonatsalze, jedoch ausgenommen die speziellen 8-Hydroxychinolinderivate, 5-Chlor-8-hydroxychinolin, 5,7-Dichlor-8-hydroxychinolin, 5-Chlor-7-jod-8-hydroxychinolin und 5,7-Dibrom-8-hydroxychinolin.

5. Ein Komplex mit Indium(III) eines ringsubstituierten Derivats von 8-Hydroxychinolin, das einen Halogensubstituenten in einer oder beiden der 5- und 7-Sellungen hat und das gegenenfalls ein oder mehrere weitere Ringsubstituenten, ausgewählt aus der Gruppe bestehend aus $C_{1-4}$-Alkyl, Carboxy und Sulfo, haben kann und die physiologisch annehmbaren Carboxylat- und Sulfonatsalze, jedoch ausgenommen das spezielle 8-Hydroxychinolinderivat, 5,7-Dibrom-8-hydroxychinolin, wobei der genannte Komplex in fester Form vorliegt.

6. Ein Komplex nach Anspruch 2, 4 oder 5, dadurch gekennzeichnet, daß der Halogensubstituent oder die Substituenten aus Chlor, Jod und Brom ausgewählt ist bzw. sind.

7. Ein Komplex nach Anspruch 6, dadurch gekennzeichnet, daß das 8-Hydroxychinolinderivat nur durch Halogensubstituenten in einer oder beiden der 5- und 7-Stellungen substituiert ist.

8. Ein Komplex nach Anspruch 2, dadurch gekennzeichnet, daß das 8-Hydroxychinolinderivat aus 5-Chlor-8-hydroxychinolin, 7-Chlor-8-hydroxychinolin, 5,7-Dichlor-8-hydroxychinolin, 5-Chlor-7-jod-8-hydroxychinolin, 5,7-Dijod-8-hydroxychinolin, 5,7-Dibrom-8-hydroxychinolin, 7-Brom-5-chlor-8-hydroxychinolin und 7-Jod-8-hydroxychinolin-5-sulfonsäure und physiologisch annehmbaren Sulfonatsalzen davon ausgewählt ist.

9. Ein Komplex nach Anspruch 4, dadurch gekennzeichnet, daß das 8-Hydroxychinolinderivat aus 7-Chlor-8-hydroxychinolin, 7-Brom-5-chlor-8-hydroxychinolin, 5,7-Dijod-8-hydroxychinolin und 7-Jod-8-hydroxychinolin-5-sulfonsäure und physiologisch annehmbaren Sulfonatsalzen davon ausgewählt ist.

10. Ein Komplex nach Anspruch 5 oder 8, dadurch gekennzeichnet, daß das 8-Hydroxychinolinderivat 5,7-Dichlor-8-hydroxychinolin ist.

11. Ein Komplex nach Anspruch 1 oder 3, dadurch gekennzeichnet, daß er einen fluorierten $C_{1-4}$-Alkylgruppensubstituenten in 2-Stellung aufweist.

12. Ein Komplex nach Anspruch 11, dadurch gekennzeichnet, daß er einen Trifluormethylsubstituenten in 2-Stellung aufweist.

13. Ein Komplex nach Anspruch 12, dadurch gekennzeichnet, daß das 8-Hydroxychinolinderivat 2-Trifluormethyl-8-hydroxychinolin ist.

14. Ein Komplex nach einem der vorstehenden Ansprüche, dadurch gekennzeichnet, daß er ein Molverhältnis von $In^{3+}$:8-Hydroxychinolinderivatanion von 1:1, 1:2 oder 1:3 enthält.

15. Ein Komplex nach Anspruch 14, dadurch gekennzeichnet, daß er ein Molverhältnis von $In^{3+}$:8-Hydroxychinolinderivatanion von 1:3 enthält.

16. Verfahren zur Herstellung eines Indium(III)-Komplexes nach Anspruch 3, dadurch gekennzeichnet, daß man das entsprechende 8-Hydroxychinolinderivat mit $In^{3+}$-Ionen umsetzt.

17. Verfahren zur Herstellung eines Indium(III)-Komplexes nach Anspruch 5, dadurch gekennzeichnet, daß man das entsprechende 8-Hydroxychinolinderivat mit $In^{3+}$-Ionen umsetzt und danach den Komplex durch Zugabe von Wasser zu dessen Lösung in einem organischen Lösungsmittel ausfällt.

18. Verfahren nach Anspruch 16 oder 17, dadurch gekennzeichnet, daß man die $In^{3+}$-Ionen durch ein Indiumtrihalogenid zur Verfügung stellt.

19. Arzneimittel, dadurch gekennzeichnet, daß es einen Indium(III)-Komplex eines ringsubstituierten Derivats von 8-Hydroxychinolin, das einen Halogensubstituenten in einer oder beiden der 5- und 7-Stellungen und/oder einen halogenierten $C_{1-4}$-Alkylgruppensubstituenten in 2-Stellung aufweist, und das gegebenenfalls ein oder mehrere weitere Ringsubstituenten, ausgewählt aus der Gruppe bestehend aus $C_{1-4}$-Alkyl, Carboxy und Sulfo, aufweisen kann und physiologisch annehmbare Carboxylat- und Sulfonatsalze zusammen mit einem physiologisch annehmbaren Verdünnungsmittel oder Träger, jedoch unter Ausschluß irgendeines Verdünnungsmittels, das nicht steril und nicht pyrogenfrei ist, enthält.

20. Mittel, das einen sterile und pyrogenfreie Lösung, Suspension oder Emulsion darstellt, zur Behandlung von bakteriellen, durch Pilze hervorgerufenen und durch Parasiten hervorgerufenen Infektionen bei Menschen oder Tieren, dadurch gekennzeichnet, daß es einen Indium(III)-Komplex nach Anspruch 19 enthält.

21. Festes Mittel zur Behandlung von bakteriellen, durch Pilze hervorgerufenen oder durch Parasiten

hervorgerufenen Infektionen bei Menschen oder Tieren, dadurch gekennzeichnet, daß es einen Indium(III)-Komplex nach Anspruch 19 enthält.

22. Festes Mittel, das für die orale Verabreichung ausgebildet ist, dadurch gekennzeichnet, daß es einen Indium(III)-Komplex nach Anspruch 19 enthält.

23. Mittel, das für die topische Anwendung zur Behandlung von bakteriellen, durch Pilze hervorgerufenen und durch Parasiten hervorgerufenen Infektionen bei Menschen oder Tieren ausgebildet ist, dadurch gekennzeichnet, daß es einen Indium(III)-Komplex nach Anspruch 19 enthält.

24. Mittel nach Anspruch 23, dadurch gekennzeichnet, daß es eine Salbe oder ein Shampoo ist.

25. Mittel nach Anspruch 23, dadurch gekennzeichnet, daß es ein verformtes Material darstellt, das so ausgebildet ist, das es in Kontakt mit der Haut bei-behalten wird.

26. Mittel nach einem der Ansprüche 19 bis 25, dadurch gekennzeichnet, daß das 8-Hydroxychinolinderivat einen Halogensubstituenten in einer oder beiden der 5- und 7-Stellungen aufweist.

27. Mittel nach Anspruch 26, dadurch gekennzeichnet, daß der Halogensubstituent oder die Halogensubstituenten aus Chlor, Jod und Brom ausgewählt ist bzw. sind.

28. Mittel nach Anspruch 27, dadurch gekennzeichnet, daß das 8-Hydroxychinolinderivat nur durch Halogensubstituenten in einer oder beiden der 5- und 7-Stellungen substituiert ist.

29. Mittel nach Anspruch 27, dadurch gekennzeichnet, daß das 8-Hydroxychinolinderivat aus 5-Chlor-8-hydroxychinolin, 7-Chlor-8-hydroxychinolin, 5,7-Dichlor-8-hydroxychinolin, 5-Chlor-7-jod-8-hydroxychinolin, 5,7-Dijod-8-hydroxychinolin, 5,7-Dibrom-8-hydroxychinolin, 7-Brom-5-chlor-8-hydroxychinolin und 7-Jod-8-hydroxychinolin-5-sulfonsäure und physiologisch annehmbaren Sulfonatsalzen davon ausgewählt ist.

30. Mittel nach Anspruch 27, dadurch gekennzeichnet, daß das 8-Hydroxychinolinderivat 5,7-Dichlor-8-hydroxychinolin ist.

31. Verwendung eines Indium(III)-Komplexes nach Anspruch 1 zur Herstellung eines Arzneimittels zur Verwendung bei der Behandlung von bakteriellen, durch Pilze hervorgerufenen und durch Parasiten hervorgerufenen Infektionen.

**Revendications**

1. Complexe avec l'indium (III) d'un dérivé de la 8-hydroxyquinoléine, substitué sur le noyau, comportant un substituant halogéno sur l'une des positions 5 et 7 ou sur les deux et/ou comportant un groupe alkyle en $C_1$ à $C_4$, halogéné, comme substituant en position 2, et qui peut éventuellement comporter un ou plusieurs substituants supplémentaires fixés sur le noyau et choisis dans l'ensemble constitué par un groupe alkyle en $C_1$ à $C_4$, un groupe carboxy, un groupe sulfo, et des sels carboxyliques et sulfoniques physiologiquement acceptables, pour servir en thérapie.

2. Complexe avec l'indium (III) d'un dérivé de la 8-hydroxyquinoléine, substitué sur le noyau et comportant un substituant halogéno sur l'une des positions 5 et 7, ou sur les deux, et qui peut éventuellement comporter un ou plusieurs substituants supplémentaires fixés sur le noyau et choisis dans l'ensemble constitué par un groupe alkyle en $C_1$—$C_4$, un groupe carboxy et un groupe sulfo, et des sels carboxyliques et sulfoniques physiologiquement acceptables, pour servir en thérapie.

3. Complexe avec l'indium (III) d'un dérivé de la 8-hydroxyquinoléine, substitué sur le noyau, comportant un substituant halogéno fixé sur l'une des positions 5 et 7, ou sur les deux, et/ou un groupe alkyle en $C_1$ à $C_4$, halogéné, comme substituant en position 2, et qui peut éventuellement comporter un ou plusieurs substituants supplémentaires fixés sur le noyau et choisis parmi l'ensemble constitué par un groupe alkyle en $C_1$ à $C_4$, un groupe carboxy et sulfo et des sels carboxyliques et sulfoniques physiologiquement acceptables, mais à l'exclusion des dérivés spécifiques de la 8-hydroxyquinoléine que sont la 5-chloro-8-hydroxyquinoléine, la 5,7-dichloro-8-hydroxyquinoléine, la 5-chloro-7-iodo-8-hydroxyquinoléine et la 5,7-dibromo-8-hydroxyquinoléine.

4. Complexe avec l'indium (III) d'un dérivé de la 8-hydroxyquinoléine, substitué sur le noyau et comportant un substituant halogéno sur l'une des positions 5 et 7 ou sur les deux, et que peut éventuellement comporter un ou plusieurs substituants supplémentaires fixés sur le noyau et choisis dans l'ensemble constitué par un groupe alkyle en $C_1$ à $C_4$, un groupe carboxy, un groupe sulfo et leurs sels carboxyliques et sulfoniques physiologiquement acceptables, mais à l'exclusion des dérivés spécifiques de la 8-hydroxyquinoléine que sont la 5-chloro-8-hydroxyquinoléine, la 5,7-dichloro-8-hydroxyquinoléine, la 5-chloro-7-iodo-8-hydroxyquinoléine et la 5,7-dibromo-8-hydroxyquinoléine.

5. Complexe avec l'indium (III) d'un dérivé de la 8-hydroxyquinoléine, substitué sur le noyau et comportant un substituant halogéno sur l'une des positions 5 et 7 ou sur les deux, et qui peut éventuellement comporter un ou plusieurs substituants supplémentaires fixés sur le noyau et choisis dans l'ensemble constitué par un groupe alkyle en $C_1$ à $C_4$, un groupe carboxyle, un groupe sulfo et leurs sels carboxyliques et sulfoniques physiologiquement acceptables, mais à l'exclusion du dérivé spécifique de la 8-hydroxyquinoléine qu'est la 5,7-dibromo-8-hydroxyquinoléine, ledit complexe étant sous forme solide.

6. Complexe selon la revendication 2, 4 ou 5, dans lequel le substituant ou les substituants halogéno est ou sont choisis parmi un substituant chloro, iodo et bromo.

7. Complexe selon la revendication 6, dans lequel le dérivé de la 8-hydroxyquinoléine ne porte comme substituant(s) que des substituants halogéno fixés sur l'une des positions 5 et 7 ou sur les deux.

8. Complexe selon la revendication 2, dans lequel le dérivé de la 8-hydroxyquinoléine est choisi parmi la 5-chloro-8-hydroxyquinoléine, la 7-chloro-8-hydroxyquinoléine, la 5,7-dichloro-8-hydroxyquinoléine, la 5-chloro-7-iodo-8-hydroxyquinoléine, la 5,7-diiodo-8-hydroxyquinoléine, la 5,7-dibromo-8-hydroxyquinoléine, la 7-bromo-5-chloro-8-hydroxyquinoléine, et l'acide 7-iodo-8-hydroxyquinoléine 5-sulfonique et ses sels sulfoniques physiologiquement acceptables.

9. Complexe selon la revendication 4, dans lequel le dérivé de la 8-hydroxyquinoléine est choisi parmi la 7-chloro-8-hydroxyquinoléine, la 7-bromo-5-chloro-8-hydroxyquinoléine, la 5,7-diiodo-8-hydroxyquinoléine et l'acide 7-iodo-8-hydroxyquinoléine 5-sulfonique et ses sels sulfoniques physiologiquement acceptables.

10. Complexe selon la revendication 5 ou 8, dans lequel le dérivé de la 8-hydroxyquinoléine et la 5,7-dichloro-8-hydroxyquinoléine.

11. Complexe selon la revendication 1 ou 3, qui comporte un groupe alkyle en $C_1$ à $C_4$, fluoré, comme substituant en position 2.

12. Complexe selon la revendication 11, qui comporte un substituant trifluorométhyle en position 2.

13. Complexe selon la revendication 12, dans lequel le dérivé de la 8-hydroxyquinoléine est la 2-trifluorométhyl-8-hydroxyquinoléine.

14. Complexe selon l'une quelconque des revendications précédentes, qui contient une proportion molaire de 1:1, 1:2 ou 1:3 de $In^{3+}$:anion dérivé de la 8-hydroxyquinoléine.

15. Complexe selon la revendication 14, qui contient une proportion molaire de 1:3 de $In^{3+}$:anion dérivé de la 8-hydroxyquinoléine.

16. Procédé pour la préparation d'un complexe d'indium (III) tel que défini à la revendication 3, qui comprend la réaction du dérivé correspondant de 8-hydroxyquinoléine avec des ions $In^{3+}$.

17. Procédé pour la préparation d'un complexe d'indium (III) tel que défini à la revendication 5, qui comprend la réaction du dérivé correspondant de la 8-hydroxyquinoléine avec des ions $In^{3+}$ puis la précipitation du complexe par l'addition d'eau à une solution de ce complexe dans un solvant organique.

18. Procédé selon la revendication 16 ou 17, dans lequel les ions $In^{3+}$ sont fournis par un trihalogénure d'indium.

19. Composition pharmaceutique qui comprend un complexe d'indium (III) d'un dérivé de substitution sur le noyau d'une 8-hydroxyquinoline comportant un substituant halogéno sur l'une des positions 5 et/7 ou sur les deux et/ou un groupe alkyle en $C_1$ à $C_4$, halogéné, comme substituant en position 2, et qui peut éventuellement comporter un ou plusieurs substituants supplémentaires du noyau, choisi(s) dans l'ensemble constitué par un groupe alkyle en $C_1$ à $C_4$, un groupe carboxy et un groupe sulfo et les sels carboxyliques et sulfoniques physiologiquement acceptables, avec un diluant, véhicule ou support physiologiquement acceptable, mais à l'exclusion de tout diluant qui n'est pas stérile et n'est pas dépourvu de pyrogènes.

20. Composition qui est une solution, suspension ou émulsion stérile et sans pyrogènes, pour le traitement d'infections bactériennes, fongiques et parasitaires des êtres humains ou des animaux, qui comprend un complexe d'indium (3) tel que défini à la revendication 19.

21. Composition solide pour le traitement d'infections bactériennes, fongiques ou parasitaires des êtres humains ou des animaux, qui comprend un complexe d'indium (3) tel que défini à la revendication 19.

22. Composition solide destinée à une administration orale, qui comprend un complexe d'indium (III) tel que défini à la revendication 19.

23. Composition destinée à une application topique pour le traitement d'infections bactériennes, fongiques et parasitaires chez les êtres humains ou les animaux, qui comprend un complexe d'indium (III) tel que défini à la revendication 19.

24. Composition selon la revendication 23, qui est une pommade, un onguent ou un shampooing.

25. Composition selon la revendication 23, qui est une matière façonnée destinée à être retenue en contact avec la peau.

26. Composition selon l'une quelconque des revendications 19 à 25, dans laquelle le dérivé de la 8-hydroxyquinoléine comporte un substituant halogéno sur l'une des positions 5 et 7 ou sur les deux.

27. Composition selon la revendication 26, dans laquelle le ou les substituants halogéno est ou sont choisis parmi les substituants chloro, iodo et bromo.

28. Composition selon la revendication 27, dans laquelle le dérivé de la 8-hydroxyquinoléine n'est substitué que par les substituants halogéno sur l'une des positions 5 et 7 ou sur les deux.

29. Composition selon la revendication 27, dans laquelle le dérivé de la 8-hydroxyquinoléine est choisi parmi la 5-chloro-8-hydroxyquinoléine, la 7-chloro-8-hydroxyquinoléine, la 5,7-dichloro-8-hydroxyquinoléine, la 5-chloro-7-iodo-8-hydroxyquinoléine, la 5,7-diiodo-8-hydroxyquinoléine, la 5,7-dibromo-8-hydroxyquinoléine, la 7-bromo-5-chloro-8-hydroxyquinoléine et l'acide 7-iodo-8-hydroxyquinoléine 5-sulfonique et ses sels sulfoniques physiologiquement acceptables.

30. Composition selon la revendication 27, dans laquelle le dérivé de la 8-hydroxyquinoléine est la 5,7-dichloro-8-hydroxyquinoléine.

31. Utilisation d'un complexe d'indium (3), selon la définition de la revendication 1, pour la fabrication d'un médicament destiné à servir au traitement d'infections bactériennes, fongiques et parasitaires.